Europäisches Patentamt

European Patent Office

Office européen des brevets

(11) Publication number: **0 299 206 B1**

# EUROPEAN PATENT SPECIFICATION

(45) Date of publication of patent specification: **10.05.95**

(51) Int. Cl.⁶: **C12P 21/02**, C12N 15/00, C12N 1/20, C12N 1/18, C12N 5/00

(21) Application number: **88109311.6**

(22) Date of filing: **10.06.88**

The file contains technical information submitted after the application was filed and not included in this specification

(54) **Recombinant human ADF.**

(30) Priority: **12.06.87 JP 146348/87**

(43) Date of publication of application:
**18.01.89 Bulletin 89/03**

(45) Publication of the grant of the patent:
**10.05.95 Bulletin 95/19**

(84) Designated Contracting States:
**DE FR GB IT**

(56) References cited:

**CHEMICAL ABSTRACTS, vol. 107, no. 7, 17th August 1987, page 602, abstract no. 57448j, Columbus, Ohio, US; & JP-A-62 19 533 (AJINOMOTO CO., INC.) 28-01-1987**

**CHEMICAL ABSTRACTS, vol. 107, no. 7, 17th August 1987, pages 601,602, abstract no. 57447h, Columbus, Ohio, US; & JP-A-62 19 532 (AJINOMOTO CO., INC.) 28-01-1987**

(73) Proprietor: **AJINOMOTO CO., INC.**
**5-8, Kyobashi 1-chome, Chuo-ku**
**Tokyo 104 (JP)**

Proprietor: **Yodoi, Junji**
**39, Kitashirakawa-Nishisenouchi-cho**
**Sakyo-ku**
**Kyoto-Shi**
**Kyoto-fu (JP)**

(72) Inventor: **Yodoi, Jyunji**
**No. 39, Kitashirajkawa**
**Nishisenouchi-cho**
**Sakyo-ku**
**Kyoto-shi**
**Kyoto-fu (JP)**
Inventor: **Tagaya, Yutaka**
**No. 14-11, Uyama-cho**
**Hirakata-ahi**
**Osaka-fu (JP)**
Inventor: **Maeda, Michiyuki**
**No. 1-19, Takanohigashibiraki-cho**
**Sakyo-ku**
**Kyoto-shi**
**Kyoto-fu (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

THE JOURNAL OF IMMUNOLOGY, vol. 140, no. 8, 15th April 1988, pages 2614-2620, The American Associaten of Immunologists, US; Y. TAGAYA et al.: "IL-2 receptor[p55]/Tac-inducing factor"

CHEMICAL ABSTRACTS, vol. 103, no. 3, 22nd July 1985, page 446, abstract no. 20981u, Columbus, Ohio, US; K. TESHIGAWARA et al.: "Adult T leukemia cells produce a lymphokine that augments interleuken 2 receptor expression", & J. MOL. CELL. IMMUNOL. 1985, 2(1), 17-26

CHEMICAL ABSTRACTS, vol. 106, no. 21, 25th May 1987, page 563, abstract no. 174419r, Columbus, Ohio, US; J. YODOI et al.: "IL-2 receptor gene regulation by cytokines: role of ATL-derived factor(s) (ADFs) in ATL", & ACTA HISTOCHEM. CYTOCHEM. 1986, 19(6), 719-33

THE JOURNAL OF IMMUNOLOGY (1986) 136: 3304-3310

Inventor: **Matsui, Hiroshi**
**No. 1-1, Suzuki-cho**
**Kawasaki-ku**
**Kawasaki-shi**
**Kanagawa-ken (JP)**
Inventor: **Kondo, Nobuo**
**No. 1-1, Suzuki-cho**
**Kawasaki-ku**
**Kawasaki-shi**
**Kanagawa-ken (JP)**
Inventor: **Hamuro, Jyunji**
**No. 1-1, Suzuki-cho**
**Kawasaki-ku**
**Kawasaki-shi**
**Kanagawa-ken (JP)**


(74) Representative: **Strehl Schübel-Hopf Groening & Partner**
**Maximilianstrasse 54**
**D-80538 München (DE)**

**Description**

The present invention relates to recombinant human ADF polypeptide, genes coding for human ADF, plasmids containing the genes and transformants transformed by the plasmids, a method of producing the objective recombinant human ADF by culturing the transformants as well as to pharmaceutical compositions and immunotherapeutic compositions containing said recombinant human ADF.

Human ADF is a substance showing activities such as cell differentiation, growth induction, etc. for blood cells such as lymphocytes and fibroblasts and is a useful substance that is widely utilizable as a therapeutic, agent for cancer and diseases associated with immunodeficiency, etc.

Description of the Background:

It is known that antigen-activated T cells express interleukin 2 receptors (hereafter simply referred to as IL2R) on their membrane surfaces. This means that T cells acquire interleukin 2 (hereafter simply referred to as IL2) responsiveness. As the mechanism, it is currently considered that T cells undergo stimulation by antigen and, at the same time, are stimulated by the soluble factor interleukin 1 (hereafter simply referred to as IL1) produced by adherent cells capable of presenting antigen, such as macrophages, etc.

As is well known, IL2 has a central function in the cellular immune response. It is reported that its function is limited not only to differentiation and growth of T cells but also extends to a very wide range such as differentiation and growth of B cells, activation of natural killer cells (NK), lymphokine-activated killer cells (LAK), monocytes, etc. At present, extensive investigations have been made on IL2 as a therapeutic agent for cancers and immunodeficiency. In order to achieve expression of the function, however, it is necessary that even with IL2, which has such a central function in the immune response, IL2 responsiveness must already have been imparted to target cells.

At present, in the case of considering immunotherapy using IL2, attention is directed not only to an immunoactive substance as a ligand but also the necessity of approaching the therapy from the point of view of responsiveness to the ligand. Thus, presence or absence of expression of the relevant receptor has been strongly desired. Considering this aspect, in the case of applying IL2 as a therapeutic agent for cancer and immunodeficiency, etc., it is considered to use IL2 in combination with an IL2 receptor expression inducing factor such as IL1, etc., in order to minimize the dosage of IL2 to eliminate side effects and strikingly enhance the effects of IL2. However, as is well known, IL1 is an endogeneous pyrogenic substance and it is impossible to administer IL1 in a native form. Thus, it has been desired to develop non-pyrogenic substances having similar function.

It has been found that human ADF is present in the culture supernatant of a T leukemia cell line (hereafter referred to as "ATL cells") established from a patient with human adult T leukemia as a substance for inducing Tac antigen (Nature, 300, 267-269 (1982), Pro. N.A.S., 80, 6957-6951 (1983), Nature, 311, 635-638 (1984)) which is considered to be IL2R (J. Immunol., 134, 1623-1630 (1985), J. Mol. Cell. Immunol., 2, 17-26 (1985)).

It has further been confirmed by subsequent investigations that human ADF of the present invention has IL1-like activity but has a structure different from that of known IL1-α and β (Nature, 315, 641 (1985), Pro. Natl. Acad. Sci., USA, 80, 7910 (1984)). Currently reported activities of IL1 are induction of IL2 production, and IL2R expression, induction of differentiation and growth of leukocyte cells, growth action on fibroblasts, etc. (J. Clin. Invest., 79, 319 (1987)). Accordingly, it can be expected that human ADF will be widely applicable as a therapeutic agent for cancer and immunodeficiency, etc. since human ADF exhibits activities such as T cell differentiation and growth of IL2 in vivo by inducing expression of IL2 and IL2R and further induces differentiation and growth of other blood cells.

As will be later described (cf. Example 3), it has been clarified that the amino acid sequence of human ADF has a structure similar to Escherichia coli thioredoxin. Human ADF also retains a structure common to the active site of Escherichia coli thioredoxin. Furthermore, human ADF also possesses a thioredoxin activity and it is thus assumed that human ADF would be the same protein as human thioredoxin. Thioredoxin is widely distributed from Escherichia coli and yeasts to higher plants and higher animals and is considered to participate in various oxidation-reduction reactions in vivo through an exchange reaction between an S-S bond and an -SH group in the thioredoxin molecule.

For reference, it is known that Escherichia coli thioredoxin functions as a hydrogen donor in the nucleic acid metabolism system of Escherichia coli, in which ribonucleotide is converted into deoxyribonucleotide. A gene of Escherichia coli thioredoxin has been isolated and its whole structure has also been clarified. Moreover, X-ray structural analysis has been carried out on the thioredoxin, whereby its steric structure is made clear and physicochemical properties are well investigated (Ann. Rev. Biochem., 54, 237-71 (1985)).

3

On the other hand, in higher animals, thioredoxins of rats, mice, cows, etc. are isolated but only partial amino acid sequences have been clarified. Neither the whole amino acid sequence, nor the genetic structure, has been clarified in any of them. In addition, an analysis of the function of thioredoxin in higher animals has not been conducted. Further, even the physiological function of thioredoxin in higher animals is still unclear.

It is reported that thioredoxin activates glucocorticoid receptor or insulin receptor through reduction in higher animals (J. Biol. Chem., 258, 13658-13664 (1983), Biochemistry, 25, 4381-4388 (1986)). It is also reported that a cell growth factor activated by 2-mercaptoethanol is present in mammalian serum (Lymphokines, 4, Academic Press) or 2-mercaptoethanol per se induces differentiation and growth of cells (J. Immunol., 121, 1899 (1987)) or 2-mercaptoethanol enhances the function of IL2 (Microbiol. Immunol., 31, 691-700 (1987)). It is suggested that reduction via an SH group as mediated by 2-mercaptoethanol would play a very important role in growth and differentiation of cells. However, 2-mercaptoethanol is not present in vivo and a substance for a reducing force donor is still unknown. It seems possible that thioredoxin could take on the role of 2-mercaptoethanol.

Rimsky, L. et al., Journal of Immunology, Vol. 136, No. 9, May 1986 describe the purification and NH$_2$-terminal amino acid sequence of a novel interleukin species derived from a human cell line. This molecule is reported to induce the proliferation of murine thymocytes in co-stimulator assays. Furthermore it is active on both human T- and B-cells, respectively, in inducing IL-2 synthesis and promoting the proliferation of anti-IgM-stimulated human peripheral blood lymphocytes. Furthermore said factor acts as a growth factor for normal human fibroblasts.

It is thus considered that this substance would play an important role not only in the immune response but also over a wide range of metabolic processes such as oxidation-reduction in vivo, for growth and differentiation and as an electron donor. Therefore, it is assumed that the substance would be very effective not only as an immunotherapeutic agent but also for inflammatory diseases accompanied by a metabolic abnormality, for example, hepatitis, etc.

In order to obtain the aforesaid human ADF, there has been hitherto adopted a method in which normal human T cells isolated from human peripheral blood are stimulated with mitogen. In another method, human ADF is produced from an human T cell line transformed by adult T leukemia virus (ATLV or refered to as HTLV-I). However, these methods involve many problems; for example, the amount of human ADF produced is low. In the case of using mitogen, harmful mitogen is a contaminant and it is difficult to remove the mitogen. Also it is necessary to supplement serum components such as fetal calf serum in T cell culture, and these supplemented proteins cannot be sufficiently separated from human ADF so that human ADF cannot be obtained in a purity sufficient to be used for medical treatment, etc.

## SUMMARY OF THE INVENTION

It is therefore an object of the present invention to clone cDNA for ADF.

It is yet another object of the present invention to produce human ADF by culturing prokaryotic or eukaryotic cells transformed by plasmids containing a gene encoding human ADF.

It is yet another object of the present invention to provide pure recombinant human ADF polypeptide produced by a genetic engineering technique.

As a result of extensive investigations to achieve the objects described above, the present inventors have produced pure recombinant human ADF in large quantities by cloning genes encoding human ADF, determining the base sequences and culturing prokaryotic or eukaryotic cells transformed by plasmids having inserted therein the ADF genes.

The above described objects are attained by a recombinant human adult T cell leukemia cell derived factor, having thioredoxin and ADF activity and having any of the following amino acid sequences:

(a)

```
             1
Val-Lys-Gln-Ile-Glu-Ser-Lys-Thr-Ala-Phe-Gln-Glu-Ala-Leu-Asp-Ala-Ala-Gly-Asp-Lys-Leu-Val-

Val-Val-Asp-Phe-Ser-Ala-Thr-Trp-Cys-Gly-Pro-

Cys-Lys-Met-Ile-Lys-Pro-Phe-Phe-His-Ser-Leu-
                          50
Ser-Glu-Lys-Tyr-Ser-Asn-Val-Ile-Phe-Leu-Glu-

Val-Asp-Val-Asp-Asp-Cys-Gln-Asp-Val-Ala-Ser-

Glu-Cys-Glu-Val-Lys-Cys-Met-Pro-Thr-Phe-Gln-

Phe-Phe-Lys-Lys-Gly-Gln-Lys-Val-Gly-Glu-Phe-

Ser-Gly-Ala-Asn-Lys-Glu-Lys-Leu-Glu-Ala-Thr-
100
Ile-Asn-Glu-Leu-Val.
```

(b) a polypeptide which in respect to (a) is deficient in one or more amino acids;

(c) a fusion polypeptide comprising a polypeptide according to (a), (b) or (c) and additional amino acids in which the additional connected amino acids do not interfere with the biological ADF activity or may be easily eliminated;

(d) a polypeptide which is an allelic derivative of a polypeptide according to any of (a), (b) or (c).

The present invention is further directed to a DNA sequence which codes for the recombinant human ADF polypeptide. According to an embodiment, this invention embraces the following base sequence (a):

```
    1
GTGAAGC AGATCGAGAG CAAGACTGCT TTTCAGGAAG

CCTTGGACGC TGCAGGTGAT AAACTTGTAG TAGTTGACTT
                        100
CTCAGCCACG TGGTGTGGGC CTTGCAAAAT GATCAAGCCT

TTCTTTCATT CCCTCTCTGA AAAGTATTCC AACGTGATAT

TCCTTGAAGT AGATGTGGAT GACTGTCAGG ATGTTGCTTC
 200
AGAGTGTGAA GTCAAATGCA TGCCAACATT CCAGTTTTTT

AAGAAGGGAC AAAAGGTGGG TGAATTTTCT GGAGCCAATA
                        300
AGGAAAAGCT TGAAGCCACC ATTAATGAAT TAGTC.
```

or the following base sequence (b):

```
1
ATGGTGAAGC AGATCGAGAG CAAGACTGCT TTTCAGGAAG

CCTTGGACGC TGCAGGTGAT AAACTTGTAG TAGTTGACTT
                100
CTCAGCCACG TGGTGTGGGC CTTGCAAAAT GATCAAGCCT

TTCTTTCATT CCCTCTCTGA AAAGTATTCC AACGTGATAT
                                    200
TCCTTGAAGT AGATGTGGAT GACTGTCAGG ATGTTGCTTC

AGAGTGTGAA GTCAAATGCA TGCCAACATT CCAGTTTTTT

AAGAAGGGAC AAAAGGTGGG TGAATTTTCT GGAGCCAATA
                300
AGGAAAAGCT TGAAGCCACC ATTAATGAAT TAGTC.
```

This invention also provides a plasmid having incorporated therein a DNA sequence as defined above, particularly a plasmid wherein the DNA sequence is incorporated operationally downstream of a promoter sequence, and a prokaryotic or eukaryotic cell transformed by this plasmid.

There is also provided a method of producing recombinant human ADF which comprises culturing in a medium the transformed prokaryotic or eukaryotic cell to produce a recombinant human ADF polypeptide and collecting said recombinant human ADF polypeptide from the medium.

Furthermore there is provided a pharmaceutical composition and a immunotherapeutic composition comprising as an active ingredient the recombinant human ADF of the present invention. The pharmaceutical composition of the present invention may be used for the treatment of inflammatory diseases.

BRIEF DESCRIPTION OF THE DRAWINGS

FIGURE 1 is a restriction enzyme map of human ADF cDNA.
FIGURE 2 shows the base sequence and the amino acids sequence of human ADF.
FIGURE 3 shows construction of plasmids pTfADF-1 and pTfADF-2 from human ADF cDNA.
FIGURE 4 shows a structure of plasmid pTfADF-2 at the ligation part between HIL-2 cDNA and human ADF cDNA.
FIGURE 5 shows construction of pAM82ADF-1.
FIGURE 6 shows construction of pCDADF-1.

DETAILED DESCRIPTION OF THE PREFERRED EMBODIMENTS

It is already known that human T cells (ATL cells) transformed by ATLV produce human ADF very efficiently. Among them, ATL-2 cell line is known to produce a particularly large quantity of human ADF.

In order to collect and identify the gene encoding human ADF, it is first necessary to collect messenger RNA (mRNA) from ATL-2 cells. When human ADF is produced in large quantities, mRNA is also produced in large quantities. Therefore, it is required that ATL-2 cells are put under the conditions in which human ADF is produced in large quantities. Accordingly, a method of establishing ATL-2 cells and culture conditions of ATL cells are first described.

Generally known methods for establishing ATL cells include the following two methods. A first method comprises collecting peripheral blood from the patient diagnosed to have ATL, harvesting leukocytes in accordance with the Ficoll method and initiating incubation in RPMI-1640 medium containing 10% fetal calf serum (FCS) supplemented with crude IL2 prepared from human peripheral blood or human spleen cells. After stable growth is shown, the crude IL2 is gradually withdrawn from the medium and several months after the initiation of incubation, a cell line that can grow and be subcultured only in RPMI-1640 medium containing 10% FCS is obtained. In order to verify that the obtained cell line is comprised of ATL cells, surface markers such as E rosette, OKT series, s-Ig, ATLA, Tac, etc. are surveyed. These antigen markers

6

EP 0 299 206 B1

are all well known and have been widely used in the clinical field.

E rosette is a survey of receptors to sheep red blood cells expressed mainly on T lymphocytes. OKT series include OKT-3, 4, 6, 8, etc. T-3 is a T cell antigen receptor-associated antigen used for identification of human peripheral blood T lymphocytes; T-4 is used for identification of the helper T lymphocyte subset; T-6 is a $\beta_2$ microglobulin-associated antigen used for identification of common thymus-derived T lymphocytes and T-8 is used for identification of a suppressor/killer T lymphocyte subset. s-Ig is an immunoglobulin on the cell surface layer and is specifically expressed on B lymphocytes. ATLA is human adult T leukemia antigen in which virus-derived protein is an antigen. Further, Tac is considered to be IL2R. Monoclonal antibodies to these antigens are all commercially available or easily accessible. Thus, FITC is bound to each monoclonal antibody and the fluorescein isothiocyanate (FITC)-bound antibody is reacted with sample cells, whereby a cell surface antigen can easily be identified. It can thus be confirmed whether cells are ATL cells or not.

A second method is transformation (in vitro) of peripheral blood using human adult T leukemia virus (ATLV) (Science, 217, 737 (1982)). This method comprises initiating incubation of both ATLV-producing ATL cells, for example, MT-2 cells, the growth capability of which has been completely abolished by irradiation with $\gamma$ rays (12,000 rad), and leukocytes obtained by treating normal human-derived peripheral blood with Ficoll in RPMI-1640 medium (containing 10% FCS) and exchanging half of the medium every 3 to 5 days. Two weeks to one month after the initiation of incubation, a cell line that can be subcultured is obtained. In case the growth is unstable, IL2 derived from, e.g., human peripheral blood, etc. is supplemented. In order to confirm that the obtained cell line is comprised of ATL cells, surface markers such as E rosette, OKT series, s-Ig, ATLA, Tac, etc. are surveyed in a manner similar to the first method.

The ATL-2 cells used in the present invention are the cell line established from peripheral blood of a patient with ATL.

Next, a method for culture of ATL cells will be described. To proliferate ATL cells, ATL cells are cultured in a medium containing fetal calf serum (FCS) to increase the count of ATL cells. At this stage, the cells may be isolated and washed to harvest mRNA. The desired mRNA may also be harvested by transferring complete synthetic medium for human ADF production free from proteins such as FCS, etc. and then further culturing.

The main components of the medium that is used to culture ATL cells may be those for commercially available media. The medium may be, for example, RPMI-1640 medium, modified Eagle's medium (MEM), Dulbecco-modified Eagle's medium (DMEM) or Click's medium. As additives for these media, there may be used, depending upon necessity, i) 20 to 250 units per 1 ml, ideally about 100 units per 1 ml of penicillin, ii) 1 to 100 $\mu$g per 1 ml, ideally about 10 $\mu$g per 1 ml of gentamycin, iii) 20 to 250 $\mu$g per 1 ml, ideally about 100 $\mu$g of streptomycin, iv) about 100 to 1000 $\mu$g per 1 ml, ideally about 300 $\mu$g per 1 ml of fresh L-glutamine, v) 1 to 3 g/l, ideally about 2 g/l of $NaHCO_3$, vi) $5 \times 10^{-4}$ to $5 \times 10^{-6}$ M, ideally $5 \times 10^{-5}$ M of 2-mercaptoethanol, etc. As the medium most suited to increase the count of ATL cells, for example, media obtained by further supplementing 1 to 30%, preferably 10% of FCS in the media described above are used.

Culture of ATL cells is performed under various environmental conditions. However, it is preferred that the ATL cell culture be kept in moisture-controlled air containing approximately 5 to 10% of carbon dioxide gas in a temperature range of approximately 35 to 37°C. As incubators, there may be used tissue culture flasks such as Falcon No. 3013 or 3025 obtained from Falcon Labware, Div. Becton, Dickinson and Co.). It is also possible to use a roller bottle or a spinner jar flask obtained from the Falcon Labware described above.

Next, identification and collection of the genes encoding human ADF will be described.

RNA is extracted and collected from the ATL-2 cells cultured under the optimum condition for producing ADF described above to prepare a cDNA library. From this library, cDNA encoding human ADF is cloned. For this, the present inventors synthesized an oligonucleotide corresponding to the amino acid partial sequence at the N-terminal of human ADF produced by ATL-2 cells. Using the thus synthesized oligonucleotide probe, clones hybridized complementarily to the cDNA described above were collected to identify human ADF cDNA. The base sequence of the thus obtained cDNA was determined in a conventional manner and the structure of the genes encoding human ADF was established. It has thus been found from this gene sequence, that human ADF is a polypeptide constructed of 104 amino acids.

At the same time, a technique capable of producing human ADF polypeptide in prokaryotic or eukaryotic cells using the cDNA obtained has also been established. That is, recombinant DNA obtained by introducing the gene encoding human ADF into vector DNA may be introduced into host cells and the obtained transformed cells may be cultured.

7

Host cells into which the recombinant DNA is to be introduced include Escherichia coli, Bacillus subtilis and other prokaryotes and Saccharomyces cerevisiae, monkey COS cells, mouse L cells, CHO cells and other eukaryotic cells.

The medium for culturing the transformed host cells and the culturing method may be a conventional medium and method.

In the case where human ADF is accumulated in the transformed host cells, human ADF can be recovered and purified in a manner that can be easily carried out by one skilled in the art. Briefly stated, the manner is as follows. After culture, cells are collected by centrifugation and suspended, if necessary, in a solution containing lysozyme or a solution containing detergents. After reaction, freezing and thawing are repeated and the cell extracts are collected. Next, by various methods such as salting out, vacuum dialysis, ultrafiltration, gel filtration chromatography, ion exchange chromatography, affinity chromatography, chromatofocusing, reversed phase chromatography, gel electrophoresis, etc., purification can be accomplished.

The activity of human ADF can be measured by the following method.

Using as an index Tac antigen expression induction of YT cells (J. Immunol., 134, 1623-1630 (1985)) which are a NK-like tumor strain in which expression of IL2R (Tac antigen) is regulatable, the activity of human ADF may be measured. Namely, $3 \times 10^5$ YT cells are cultured for 24 hours by passing 5% $CO_2$ air in the presence of a sample. After washing twice with Hank's balanced solution containing 1% FCS and 0.1% $NaN_3$, the system is reacted with anti-Tac antibody having bound thereto FITC (fluorescein isothiocyanate) as a fluorescing substance for 30 minutes at 40°C to stain the cells. As a negative control, FITC-goat anti-mouse Ig antibody is used. The fluorescence intensity of the stained cells is measured by flow cytometry (Spectrum III-Ortho Pharmaceutical Co., NJ) to determine a rate of positive response. The rate is converted into the activity of human ADF.

On the other hand, the activity of thioredoxin may be measured by the following method. Using as an index a rate of reduction of the S-S bond of insulin by reduced thioredoxin, the activity of thioredoxin can be measured (J.B.C., 254, 9627-9632 (1979)). Namely, 1 mg/ml of insulin (manufactured by Sigma Co., Ltd.), 0.1M potassium phosphate buffer (pH 6.5), 2 mM EDTA and a sample are mixed and 0.3 to 5 mM dithiothreitol (DTT) is added thereto to initiate the reaction. The change of absorbance at 650 nm accompanied by the reduction of insulin is recorded and converted into the activity of thioredoxin.

The whole base sequence of human ADF which is effective for treatment of cancer, immunodeficiency and inflammatory diseases such as hepatitis or the like has been determined for the first time and at the same time, the method of producing pure recombinant human ADF using prokaryotes transformed by plasmids containing the genes has also been established according to the present invention. As such, recombinant human ADF produced by genetic engineering is free from the contamination with viruses as compared to human ADF produced by conventional culture of ATL cells and is extremely useful.

The present invention is also intended to cover amino acid sequences which are the same as that of amino acid sequence I disclosed herein except for very minor changes in specific amino acids. For example, one or a small number (e.g., 2-5) conservative amino acid changes which result in an amino acid sequence having human ADF activity, as measured by the method disclosed herein, are included within the scope of the present invention. Thus, for example, a neutral amino acid may be exchanged for another neutral amino acid. Glutamic acids, for example, could be exchanged for aspartic acids. Another change which is envisioned is between a cysteine, and, for example, a serine. As long as the resulting polypeptide retains significant human ADF activity, e.g., at least 50% of the activity of the polypeptide having amino acid sequence I as determined by the assay disclosed in this application, the polypeptide is considered to be within the scope of the present invention. Genes encoding these polypeptides are also within the scope of this invention. These modified human ADFs could be prepared by methods known in the art, such as site-directed mutagenesis, as described in Synthesis and Applications of DNA and RNA, edited by Saran A. Narang (Academic Press, Inc., New York, N.Y., 1987). Plasmids containing these modified genes and microorganisms transformed with the plasmids are also within the scope of the present invention.

Hereafter the present invention will be described with reference to the following examples which are not intended to be limiting of the present invention, except where so indicated.

EXAMPLES

Example 1: Establishment of human ADF-producing ATL cells

As human ADF-producing ATL cells, there is, for example, the ATL-2 cell line. The ATL-2 cell line was established from the peripheral blood of a 61-year old male who was diagnosed as having adult T cell leukemia (ATL). The peripheral blood collected from the patient at the initiation of culture was judged to be comprised of leukemia cells based on the hetero type of cell nucleus. The surface character of the leukemia cells was E rosette ( + ), OKT-3 ( + ), 4 ( + ), 6 (-), 8 (-) and la ( + ), and ATLA antibody in serum was positive. As the medium, there was used RPMI-1640 medium (containing 10% FCS) supplemented with human peripheral blood or spleen cell-derived crude IL2. The cells showed stable growth for 3 months after the initiation of culture and it became possible to subculture every 3 to 4 days. Further, IL2 dependency was gradually lost at about 10 months after the initiation of culture and, even though IL2 was not supplemented, it became possible to subculture in 10% FCS-containing RPMI-1640 medium alone. The subculture has been continued to date over a period of 5 years and 8 months. The character of ATL-2 cells was E rosette ( + ), OKT-3 (-), 4 ( + ), 8 (-), Tac ( + ) and Slg (-). A number of C-type retro virus particles (ATLV) were observed by an electron microscope.

ATL cell lines having various surface characteristics are established from the patient with ATL but most human ADF-producing cell lines are of OKT-4 ( + ) type, namely, the helper phenotype, like ATL-2 cells.

Example 2

The following partial amino acid sequence of the N-terminal of human ADF has already been determined (Published Unexamined Japanese Patent Application No. 62-19532):

Val-Lys-Gln-Ile-Glu-Ser-Lys-Thr-Ala-Phe-Gln-Glu-Ala-Leu-Asp-Ala-Ala.

Several kinds of oligonucleotides encoding the partial amino acid sequence of this human ADF at the N-terminal thereof were synthesized as described below.

Synthesis of oligonucleotides was performed by a nucleotide binding reaction in accordance with the phosphinic acid triester method using DNA Synthesizer Model 380A manufactured by Applied Bio System Co., Ltd. using silica gel as a solid phase carrier. After protective groups were removed in a conventional manner, the objective oligonucleotides were purified using an acetonitrile gradient by $C_{18}$ reversed phase column HPLC.

Combinations of the N-terminal amino acid fragment 1 (Val-Lys-Gln-Ile-Glu) and the corresponding nucleotide sequence are as follows.

```
           1     2     3     4     5
          Val   Lys   Gln   Ile   Glu
     5'   GTT   AAA   CAA   ATT   GA    3'   ....   N-1
           C     G     G     C

          GTT   AAA   CAA   ATA   GA         ....   N-2
           C     G     G

          CTA   AAA   CAA   ATT   GA         ....   N-3
           G     G     G     C

          GTA   AAA   CAA   ATA   GA         ....   N-4
           G     G     G

                                     (48 mixtures in total)
```

Combinations of the N-terminal amino acid fragment 2 (Lys-Thr-Ala-Phe-Gln) and the corresponding nucleotide sequence are as follows.

```
        7     8     9    10    11
  5'   Lys   Thr   Ala   Phe   Gln   3'
       AAA   ACT   GCT   TTT   CA         ....   N-5
        G     C     C     C

       AAA   ACT   GCA   TTT   CA         ....   N-6
        G     C     G     C

       AAA   ACA   GCT   TTT   CA         ....   N-7
        G     G     C     C

       AAA   ACA   GCA   TTT   CA         ....   N-8
        G     G     G     C
```

                    (16 mixtures, respectively)

Combinations of the N-terminal amino acid fragment 3 (Ala-Phe-Gln-Glu-Ala) and the corresponding nucleotide sequence are as follows.

```
        7     8     9    10    11
  3'   Ala   Phe   Gln   Glu   Ala   5'
       CGA   AAA   GTT   CTC   CG          ....   N-9
        T     G     C     T
        G
        C
```

                    (32 mixtures)

Combinations of the N-end amino acid fragment 4

```
   1    2    3    4    5    6    7    8    9   10   11   12   13
```

   (Val-Lys-Gln-Ile-Glu-Ser-Lys-Thr-Ala-Phe-Gln-Glu-Ala) and the corresponding nucleotide sequence were assumed as follows, taking codon usage, etc., into account.

```
   1    2    3    4    5    6    7    8    9   10   11   12   13
  Val  Lys  Gln  Ile  Glu  Ser  Lys  Thr  Ala  Phe  Gln  Glu  Ala

  5'                                                             3'
  GC  TTC  TTG  GAA  GGC  GGT  CTT  AGA  TTC  GAT  TTG  CTT  GAC
                                      GCT
                                                    ...N-10
```

                    (2 mixtures)

Using the oligonucleotide probes thus prepared, the objective human ADF genes were obtained as described below.

Example 3

(a) ATL-2 having a cell number of 2 x $10^5$/ml was inoculated on 1 liter of 20% FCS-containing RPMI-1640 medium (containing 2 mM glutamine, 100 units/ml penicillin, 100 $\mu$g/ml streptomycin and 16 mM NaHCO$_3$) charged in a plastic roller bottle (Falcon #3027) (hereafter referred to as a roller) of a 2 liter volume. While rotating at 8 rpm, culture was performed for 3 days at 37°C. After culturing, the culture was centrifuged to collect the cells. After washing the cells twice with PBS, the cells (1.5 x $10^9$ cells) were suspended in 800 ml of ribonucleoside-vanadyl complex (10 mM)-containing RSB solution (10 mM Tris-HCl, pH 7.5, 10 mM NaCl, 1.5 mM MgCl$_2$). Next NP-40 was added in a 0.05% concentration followed by gentle agitation. Thereafter, the system was centrifuged at 3000 rpm for 5 minutes to remove cell debris containing nuclei. After SDS (final concentration, 0.5%) and EDTA (final concentration, 5 mM) were added to the supernatant, an equal amount of phenol was immediately added to the system to extract cytoplasmic RNA. The extraction with phenol was repeated 3 times in total. Then, RNA was precipitated with 2 volumes of ethanol. The precipitates were collected by centrifugation and dissolved in 10 mM Tris-HCl, pH 7.5. The amount of RNA thus obtained from ATL-2 cells was 25 mg.

Next, in order to obtain mRNA from this RNA, column chromatography was performed using oligo-(dT)-cellulose (Pharmacia Biochemicals, Type 7). Adsorption was carried out by dissolving RNA in 20 mM Tris-HCl, pH 7.5, 0.5 M NaCl, 1 mM EDTA and 0.5% SDS solution. After washing with buffer (20 mM Tris-HCl, pH 7.5, 0.5 M NaCl, 1 mM EDTA), elution was conducted by eluting mRNA with water and 10 mM Tris-HCl (pH 7.5) one after the other. As a result, the amount of mRNA eluted was 480 $\mu$g.

(b) Double strand a cDNA was prepared using 5 $\mu$g of mRNA prepared in (a). In accordance with the method of GUBLER, U. and HOFFMAN, B.J. (Gene, 25, 263 (1983)), double stranded cDNA was prepared following the protocol of Amersham using a cDNA synthesis kit (manufactured by Amersham Co., Ltd.). That is, single strand cDNA was synthesized from mRNA by reverse transcriptase. Using hybrid of mRNA and cDNA as a substrate, a nick and gap were formed on the RNA chain utilizing Escherichia coli ribonuclease H. Further, DNA was replaced with mRNA by Escherichia coli DNA polymerase I through a nick translation type reaction to produce double strand DNA. A small overhang located at the 3'-end was removed by T4 DNA polymerase to produce double strand cDNA. Finally obtained double strand cDNA was 1.48 $\mu$g.

(c) A cDNA cloning system using λgt 10 phage of Amasham was adopted for preparing a cDNA library. In order to bind cDNA obtained in (b) to the arm of λgt 10 phage DNA vector, EcoR I cleavage site linker was added to both ends of cDNA. That is, in order to first inactivate the EcoR I site on cDNA, methylation was performed by EcoR I methylase. 0.8 $\mu$g of cDNA and 200 units of EcoR I methylase were reacted at 37°C for 60 minutes and further at 70°C for 10 minutes.

Next, EcoR I linker was added to cDNA modified by EcoR I methylase using T4 DNA ligase. The reaction was carried out at 15°C overnight and then at 70°C for 10 minutes.

Then, cDNA bearing EcoR I linkers at both ends was cleaved with EcoR I and cDNA was separated from small fragments caused by EcoR I by gel filtration. Thus, cDNA having cohesive ends of EcoR I at both ends thereof was prepared.

(d) Next, in order to ligate this cDNA with the arm of λgt 10 phage vector DNA, cDNA was reacted with λgt 10 arm at 15°C overnight in the presence of T4 DNA ligase. Thus, λgt 10 vector DNA arm ligated with cDNA was prepared.

Next, Extract A and Extract B of the cDNA cloning system kit (manufactured by Amersham) were added thereto to perform in vitro packaging, whereby mature λgt 10 phage particles having incorporated ATL-2-derived cDNA thereinto were prepared. The phage having incorporated this cDNA thereinto can easily be replicated in Escherichia coli, shows lytic activity and forms plaque. However, phage having incorporated no cDNA therein cannot be replicated. Thus, only the phage having the cDNA incorporated therein can be efficiently selected.

Titration of λgt 10 phage particles having cDNA incorporated therein was performed; a phage solution capable of approximately 1,000,000 plaques could be prepared from 0.15 $\mu$g of cDNA.

(e) The obtained phage solution was diluted with SM buffer (NaCl 5.8 g, MgSO$_4$.7H$_2$O 2 g, 1M Tris-HCl (pH 7.5) 50 ml, 2% gelatin 5 ml/l) to make the whole volume 3 ml. The phage dilution was mixed with 6 ml of E. coli NM 514 strain which had been cultured overnight in liquid medium containing 0.2% maltose. The mixture was allowed to stand at 37°C for 20 minutes. The mixture of 30 $\mu$l each was mixed with 7 ml of LB medium containing 0.7% agarose which had been kept at 50°C. The mixture was put in layers on a Petri dish having a 15 cm diameter in which LB agar medium had previously been charged, followed by incubation at 37°C overnight. Approximately 30,000 or more plaques were obtained in 30 plates, respectively. The plaques covered the almost entire areas of the agar surface. The phage in the

11

plaques was adsorbed to a nitrocellulose filter by being brought into contact with the filter.

Next, the phage-adsorbed filter was treated with 0.5 N NaOH and neutralized with 0.5 M Tris-HCl (pH 7.5). After rinsing with 2 x SSC (0.3 M NaCl, 0.03 M sodium citrate), the filter was air dried and treated at 80°C for 2 hours thereby to immobilize the phage DNA onto the filter. The DNA-immobilized filter was treated at 65°C twice with 5 x SSC and 0.1% SDS solution followed by prehybridization with a solution containing 5 x SSC, 0.1% SDS, 1 x Denhardt solution (0.2% Ficoll, 0.2% polyvinyl pyrrolidone, 0.2% bovine serum albumin) and 100 $\mu$g/ml modified salmon sperm DNA at 37°C overnight. Next, hybridization was performed at 50°C overnight with a solution obtained by 1,000,000 cpm/ml of probe N-10 (Example 2) having the 5′-end labelled by kination to 5 x SSC, 0.1% SDS and 1 x Denhardt solution. Thereafter, the system was washed at 50°C several times with 5 x SSC and 0.1% SDS solution. Then, the filter was air dried and subjected to autoradiography.

As a result, 75 positive plaques were obtained. The filter was further washed at 55°C several times and subjected to autoradiography overnight so that the positive plaques were narrowed down to 20 strains. These plaques were intermingled with other plaques and not pure. Therefore, with respect to the 20 plaques, the plaques were scraped off from the positive one. The phage was diluted and again spread over plates together with E. coli NM 514 to make them single plaques as positive plaques. Phage was prepared from isolated positive plaques.

(f) A small scale preparation method of phage is described below. The positive plaques were scraped off and suspended in 1 ml of SM buffer to prepare a phage solution. 0.5 ml of this was mixed with 1 ml of a culture solution of NM 514 cultured in LB medium supplemented with 0.2% maltose. The mixture was allowed to stand at 37°C for 20 minutes. Next, 4 ml of LB medium was added to the mixture followed by shaking at 37°C for 4 hours. Then, 100 $\mu$l of chloroform was added to the mixture and it was further shaken at 37°C for 15 minutes to thereby completely lyse the cells. The culture solution was centrifuged to remove cellular debris and to make a phage solution. One microliter each of 20 kinds of phage solutions was added to a nitrocellulose filter and denatured with 0.5 N NaOH. Then, the system was neutralized with 0.5 M Tris-HCl (pH 7.5), rinsed with 2 x SSC and treated at 80°C for 2 hours to immobilize DNA. The filter was hybridized with N-1 through 4, N-5 through 8 and N-9 using the probes prepared in Example 2. All of the probes were hybridized at 28°C with respect to 18 strains except for #3 and #14 of the phage DNA number.

(g) Thus, large quantities of phage were prepared with respect to #20 which hybridized with all probes, by the following method. Firstly, isolated plaques were scraped and suspended in 1 ml of SM buffer. Then, the phage number in the suspension was previously calculated by spreading together with E coli NM 514 and forming plaques. Next, plaques were formed by spreading the suspension onto a plate having a diameter of 15 cm together with E. coli NM 514 by calculating in such a manner that 2 x 10$^5$ plaques appeared on the plate. The plaques spread mostly toward the front surface but did not adopt the form of plaques. This was eluted out with 10 ml of SM buffer and phages were collected. Debris of the cells was removed by centrifugation. Next, 1 mg/ml of DNase and RNase were added to the supernatant. After allowing to stand at 37°C for an hour, an equal amount of 20% polyethylene glycol and 2.5 M NaCl solution were added thereto. The mixture was thoroughly mixed and allowed to stand at 0°C for 2 hours. The precipitates obtained by centrifugation were dissolved in 2 ml of SM buffer. By extraction with phenol 3 times and treatment with chloroform 3 times, the phage protein was separated from DNA. To the aqueous phase was added 1/10 volume of 3 M NaOAc and a 2-fold amount of cold ethanol was added thereto. After cooling to -80°C, ethanol precipitates were recovered by centrifugation. The precipitates were rinsed with cold ethanol and dissolved in water to make a phage DNA standard.

(h) This #20 phage DNA was cleaved with EcoR I to give a cDNA insert fragment having approximately 600 base pairs. This fragment was recovered from agarose gel and labelled by nick translation, which was hybridized as a probe with the filter prepared in (f) above. The probe was hybridized with the phage of 18 strains except for #3 and #14. Namely, it has made clear that the 18 strains except for #3 and #14 had all homologous cDNA genes.

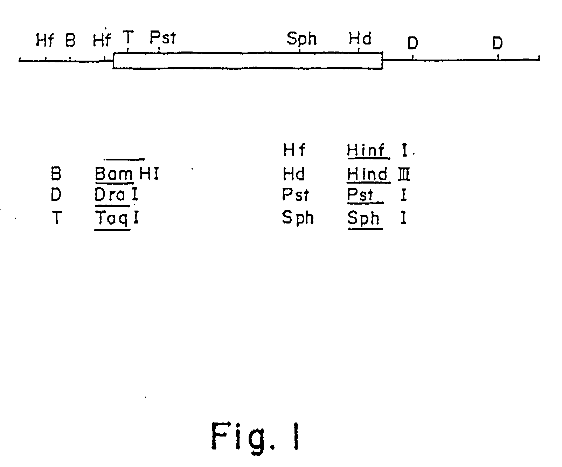

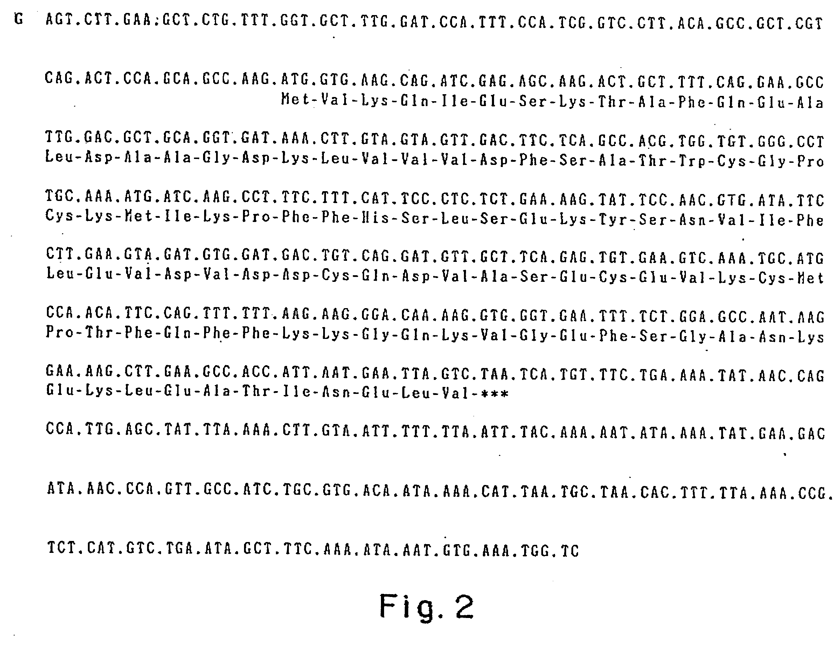

Thus, a restriction enzyme map and base sequence were determined with cDNA of #20. The base sequence was determined by the dideoxynucleotide chain termination method (F. Sanger et al., Proc. Natl. Acd. Sci., U.S.A., 74, 5463 (1977)) using M13 phage (J. Messing et al., Gene, 19, 269 (1982)). The determined base sequence and amino acid sequence are shown in FIGURE 1. The restriction enzyme map is shown in FIGURE 2.

The thus determined amino acid sequence accurately contains the human ADF N-terminal amino acid partial sequence structure shown in Example 1. From this, it is apparent that this cDNA of #20 is the objective human ADF gene.

The N-terminal of human ADF polypeptide produced by ATL cells is Val. This is considered to be because Met translated by initiation codon ATG would be cleaved during the course of secreting Met from the cells. However, the polypeptide having Met added at the N-terminal also shows equivalent human ADF activity and therefore, in the present invention, polypeptides at the N-terminal of which Met has been added or has not been added are both treated as human ADF.

Further using a computer, homology of human ADF to other known proteins was examined. As shown in Table 1, it is noted that human-ADF shows homology to Escherichia coli and rat-derived thioredoxins and in particular, the homology was high in and around the active site of thioredoxin. The active site of thioredoxin is CYS GLY PRO CYS at columns 3 and 4 in Table 1.

```
    Table 1. Comparison in N-Terminal Amino Acid Sequence

            between Human ADF and Thioredoxin


    a:     Val Lys Gln Ile Glu Ser Lys Thr Ala --- ---

    b:     Val Lys Leu Ile Asn XXX Lys Glu Ala --- ---

    c: Ser Asp Lys Ile Ile His Leu --- Thr Asp Asp Ser


       Phe Gln Glu Ala Leu Asp --- Ala Ala Gly Asp Lys

       Phe Gln Glu Ala Leu Ala --- Ala Ala Gly Asp XXX

       Phe Asp Thr Asp Leu Val Lys Ala Asp Gly Ala Ile
```

```
Leu Val Val Val Asp Phe Ser Ala Thr Trp CYS GLY

Leu Val Val Val Asp Phe XXX Ala XXX Trp CYS GLY

Leu Val --- --- Asp Phe Trp Ala Glu Trp CYS GLY



PRO CYS Lys Met Ile  Lys Pro Phe Phe ---

PRO CYS Lys Met Gln  XXX Pro XXX Phe ---

PRO CYS Lys Met Ile  Ala Pro Ile Leu ---
```

```
(Notes)     a :  human ADF

            b :  Rat Novikoff hepatoma thioredoxin

                 (Thioredoxins Structure and

                 Function, 1981, pp. 1-288, CNRS)

            c :  Escherichia coli thioredoxin (Ann.

                 Rev. Biochem., 1985, 54, 237-271)

           XXX :  unidentified amino acid
```

Example 4 Production of anti-ADF antibody

Based on the determined human ADF amino acid sequence, 10 residues from the N-terminal:

```
  1   2   3   4.  5   6   7   8   9   10
Val-Lys-Gln-Ile-Glu-Ser-Lys-Thr-Ala-Phe
```

16 residues from the 34th residue:

```
 34  35  36  37  38  39  40  41  42  43  44  45  46  47  48  49
Cys-Lys-Met-Ile-Lys-Pro-Phe-Phe-His-Ser-Leu-Ser-Glu-Lys-Tyr-Ser
```

and 28 residues from the C-terminal:

```
 77  78  79  80  81  82  83  84  85  86  87  88  89  90  91  92
Gln-Phe-Phe-Lys-Lys-Gly-Gln-Lys-Val-Gly-Glu-Phe-Ser-Gly-Ala-Asn-

 93  94  95  96  97  98  99 100 101 102 103 104
Lys-Glu-Lys-Leu-Glu-Ala-Thr-Ile-Asn-Glu-Leu-Val
```

were selected and synthetic peptides were prepared using a Peptide Synthesizer (Applied Bio Systems), respectively. Next, each synthetic peptide was conjugated with bovine serum albumin as a carrier in a conventional manner. Thereafter, the conjugate was immunized in a rabbit together with Adjuvant Complete/Incomplete Freund (manufactured by DIFCO) to obtain anti-ADF antiserum. Using the obtained antiserum, immunoblotting was performed. Any of the obtained 3 antisera could recognize purified human ADF protein.

Example 5 Construction of recombinant DNAs for expression of human ADF (prokaryote)

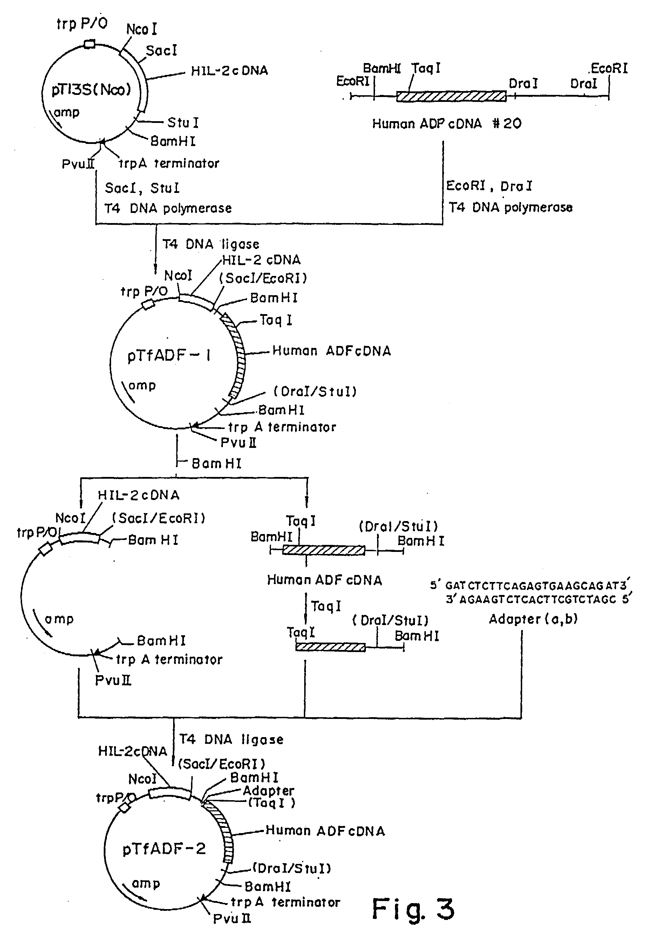

Using plasmid pT13S (Nco) ("Genetic Engineering in Medical Science" edited by Masami Muramatsu, page 146 (1987) published by Gendai Kagaku Publishing Co., as an extra edition), #20 cDNA obtained in Example 3 and synthetic adaptor DNA (a: 5′GATCTC TTC AGA GTA AAG CAG AT 3′, 23-mer, b: 5′ CGAT CTG CTT CAC TCT GAA GA3′, 21-mer), recombinant DNAs (pTfADF-1, pTfADF-2) producing human ADF-fused protein (ΔHIL-2-ADF) with the partial N-terminal of human interleukin 2 (HIL-2) were constructed (FIGURE 3).

(a) Plasmid pT13S (Nco) was cleaved with restriction enzymes Sac I and Stu I. After a large fragment was isolated and purified by agarose gel electrophoresis, the ends were made blunt with T4 DNA polymerase. On the other hand, #20 cDNA was cleaved with restriction enzymes EcoR I and Dra I. After a DNA fragment containing human ADF cDNA insert was isolated and purified by agarose gel electrophoresis, the ends were likewise made blunt with T4 DNA polymerase.

The thus prepared pT13S (Nco) fragment containing tryptophan promoter/operator and the EcoR I-Dra I fragment containing human ADF cDNA were mixed with each other and ligated using T4 DNA ligase. Thereafter, the obtained recombinant DNA was introduced into E. coli HB 101 strain and an ampicillin-resistant strain was selected.

Plasmid DNA was extracted from the obtained strain and a cleavage test was conducted with a restriction enzyme, whereby a bacterium (pTfADF-1/HB 101) bearing plasmid pTfADF-1 having inserted therein the EcoRI-Dra I fragment of human ADF cDNA downstream the Sac I site of pT13S Nco in the positive direction was selected.

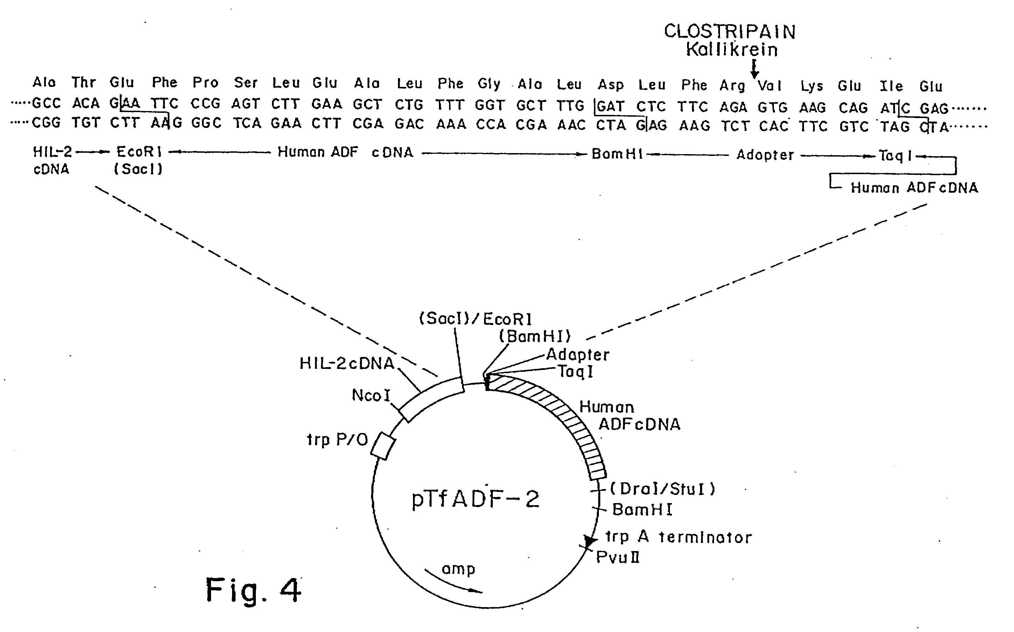

(b) Next, as shown in FIGURES 3 and 4, plasmid pTfADF-1 was cleaved with restriction enzyme BamH I. Large and small DNA fragments were isolated and purified by agarose gel electrophoresis, respectively. Further, the small fragment was cleaved with restriction enzyme Taq I to obtain the Taq I-BamH I fragment (- 350 bp).

The BamH I-cleaved large fragment of pTfADF-1 and the Taq I-BamH I fragment as well as synthetic DNA a and b fragments were mixed with each other. After ligating them using T4 DNA ligase, the obtained recombinant DNA was introduced into E. coli HB 101 strain and ampicillin-resistant strains were selected.

From the obtained strains, a strain having plasmid DNA capable of hybridizing with synthetic DNA by the colony hybridization method (cf. Example 3) was selected. Plasmid DNA was extracted from the obtained strain and a cleavage test was conducted with a restriction enzyme, whereby an E. coli HB101 (FERM BP-1885) bearing plasmid pTfADF-2 having inserted therein the Taq I-BamH I fragment downstream from the tryptophan promoter/operator via the synthetic adaptor in the positive direction was selected.

Example 6 Production of human ADF protein using E. coli as host

(a) E. coli HB 101 strain (FERM BP-1885) having plasmid pTfADF-2 was allowed to grow at 37°C overnight in 10 ml of L medium (1% bactotrypton, 0.5% yeast extract, 0.5% NaCl, 0.1% glucose, pH 7.5) containing 25 $\mu$g/ml streptomycin and 25 $\mu$g/ml ampicillin. Then, 5 ml of the culture suspension was inoculated in 100 ml of M9 Casamino acid medium (0.6% $Na_2HPO_4.12H_2O$, 0.3% $KH_2PO_4$, 0.05% NaCl, 0.1% $NH_4Cl$, 0.05% $MgSO_4.H_2O$, 0.00147% $CaCl_2$, 0.2% glucose, 0.2% casamino acid, 0.02% L-leucine, 0.02% L-proline, 0.0002% thiamine hydrochloride, 100 $\mu$g/ml ampicillin, 25 $\mu$g/ml streptomycin, pH 7.4) followed by culture at 28°C for 3 hours. Then, 3-indole acrylic acid was added in 25 $\mu$g/ml. The mixture was subjected to culture induction at 23°C for 21 hours. After culture, the cells were observed with a microscope (1000 x), whereby the formation of inclusion bodies were confirmed in the cells.

The cells were collected by centrifugation and suspended in 20 ml of 50 mM Tris-HCl buffer (pH 7.5) and, 20 mg of lysozyme and 5 ml of 0.5 M EDTA were added to the suspension. After agitation, the mixture was allowed to stand for 30 minutes on ice. Then, the cells were homogenized with an ultrasonic homogenizer (in ice, 15 minutes) and centrifuged at 10,000 rpm for 5 minutes to recover the inclusion bodies.

The inclusion bodies were suspended in 5 ml of 0.1 M Tris-HCl buffer (pH 7.5) containing 8M urea and 5 mM dithiothreitol (DTT). By allowing to stand at room temperature for 8 hours, the inclusion bodies were solubilized. A part of the solubilized inclusion bodies was analyzed by SDS-polyacrylamide gel electrophoresis and an immunoblotting method. It was confirmed that approximately 50% of the whole protein in the inclusion bodies was human IL-2-fused human ADF protein, namely, ΔHIL-2-ADF, having a molecular weight of about 21,000. The yield was about 2 mg. ΔHIL-2-ADF has the following amino acid sequence.

```
Met-Ala-Pro-Thr-Ser-Ser-Ser-Thr-Lys-Lys-Thr-Gln-Leu-

Gln-Leu-Glu-His-Leu-Leu-Leu-Asp-Leu-Gln-Met-

Ile-Leu-Asn-Gly-Ile-Asn-Asn-Tyr-Lys-Asn-Pro-

Lys-Leu-Thr-Arg-Met-Leu-Thr-Phe-Lys-Phe-Tyr-

Met-Pro-Lys-Lys-Ala-Thr-Glu-Phe-Pro-Ser-Leu-

Glu-Ala-Leu-Phe-Gly-Ala-Leu-Asp-Leu-Phe-Arg-

Val-Lys-Gln-Ile-Glu-Ser-Lys-Thr-Ala-Phe-Gln-Glu-Ala-Leu-Asp-Ala-Ala-Gly-Asp-Lys-Leu-Val-

Val-Val-Asp-Phe-Ser-Ala-Thr-Trp-Cys-Gly-Pro-

Cys-Lys-Met-Ile-Lys-Pro-Phe-Phe-His-Ser-Leu-

Ser-Glu-Lys-Tyr-Ser-Asn-Val-Ile-Phe-Leu-Glu-

Val-Asp-Val-Asp-Asp-Cys-Gln-Asp-Val-Ala-Ser-

Glu-Cys-Glu-Val-Lys-Cys-Met-Pro-Thr-Phe-Gln-

Phe-Phe-Lys-Lys-Gly-Gln-Lys-Val-Gly-Glu-Phe-

Ser-Gly-Ala-Asn-Lys-Glu-Lys-Leu-Glu-Ala-Thr-

Ile-Asn-Glu-Leu-Val.
```

(b) Cleavage with clostripain

In ΔHIL-2-ADF, the gene is constructed so as to include the amino-acid sequence of -Phe-Arg- at the conjunction part between the human IL2 part and human ADF (FIGURE 4). Using clostripain or kallikrein, the peptide bond at the C end of Arg in the sequence described above is cleaved to convert ΔHIL-2-ADF into human ADF protein having the native amino acid sequence.

The solubilized inclusion bodies, 5 ml, (containing 2 mg of HIL-2-ADF) was diluted 2-fold with distilled water. After CaCl₂ was added to the dilution in a final concentration of 0.1 M, 20 units of clostripain (manufactured by Sigma Co.) was added to the mixture to conduct a cleavage reaction at room temperature for 8 hours.

Using a part of the reaction solution, analysis was performed by SDS-polyacrylamide gel electrophoresis and immunoblotting. It was confirmed that more than 80% of ΔHIL-2-ADF was converted into natural human ADF protein having a molecular weight of about 13,000. It is considered that human ADF protein has the amino acid sequence described below.

```
¹Val-Lys-Gln-Ile-Glu-Ser-Lys-Thr-Ala-Phe-Gln-Glu-Ala-Leu-Asp-Ala-Ala-Gly-Asp-Lys-Leu-Val-

Val-Val-Asp-Phe-Ser-Ala-Thr-Trp-Cys-Gly-Pro-

Cys-Lys-Met-Ile-Lys-Pro-Phe-Phe-His-Ser-Leu-
                                    50
Ser-Glu-Lys-Tyr-Ser-Asn-Val-Ile-Phe-Leu-Glu-

Val-Asp-Val-Asp-Asp-Cys-Gln-Asp-Val-Ala-Ser-

Glu-Cys-Glu-Val-Lys-Cys-Met-Pro-Thr-Phe-Gln-

Phe-Phe-Lys-Lys-Gly-Gln-Lys-Val-Gly-Glu-Phe-

Ser-Gly-Ala-Asn-Lys-Glu-Lys-Leu-Glu-Ala-Thr-
100
  Ile-Asn-Glu-Leu-Val.
```

(c) Purification of recombinant human ADF

The human ADF solution obtained in (b) was applied on Mono Q HR 5/5 anionic ion exchange column (manufactured by Pharmacia) equilibrated with 4 M urea-2.5 mM DTT-50 mM Tris-HCl buffer (pH 7.5). Then, protein was eluted out by linear gradient elution (7.5 mM/min, flow rate of 0.5 ml/min) of NaCl.

The column operation was performed using Fast Protein Liquid Chromatography FPLC System (manufactured by Pharmacia).

The fraction containing human ADF protein eluted at the NaCl concentration of 100-200 mM was applied on $C_8$ reversed phase chromatography column (A-221-$C_8$, Yamamura Chemical Laboratories Co., Ltd., Japan) equilibrated with 0.1% trifluoroacetic acid (TFA). Thereafter, the protein was eluted out by linear gradient (1%/min, flow rate of 0.5 ml/min) of isopropyl alcohol containing 0.1% TFA.

The column operation was performed using high performance liquid chromatography, HPLC System (manufactured by Hitachi Co., Ltd.). The human ADF protein was eluted out at the isopropyl alcohol concentration of 40%.

Approximately 100 µg of purified sample was obtained. As the result of analysis by the SDS polyacrylamide gel electrophoresis and silver staining (manufactured by Nakarai ChemiCal CO., Ltd.), it was confirmed that the sample was a homogeneous protein having a molecular weight of about 13,000. Further the sample had both ADF activity (specific activity, 5 x 10⁶ U/mg) and thioredoxin activity (specific activity, 5 U/mg).

Example 7 Construction of recombinant DNA for expression of human ADF in yeast

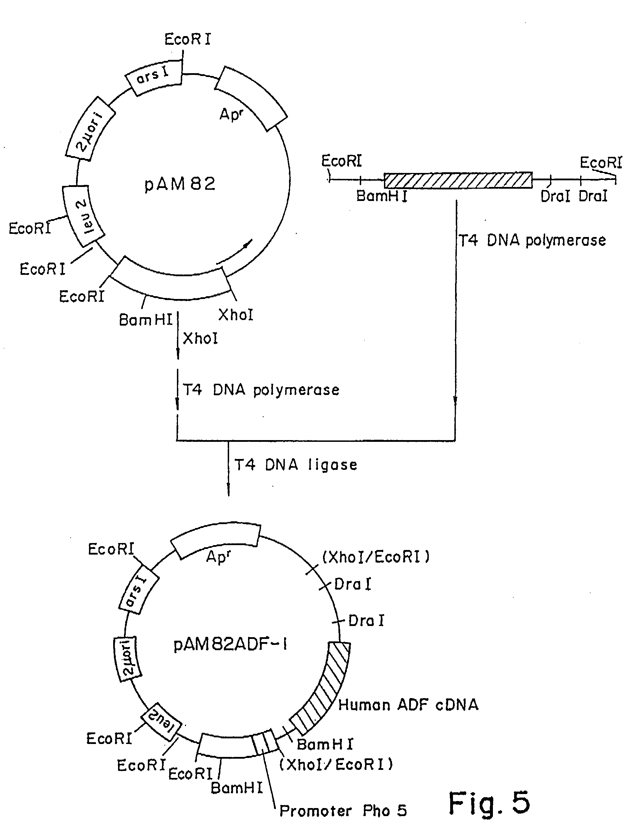

Using yeast-Escherichia coli shuttle vector pAM82 (A. Miyanohara et al., Proc. Natl. Acad. Sci., U.S.A., 80, 1-5 (1983)) and #20 cDNA obtained in Example 3, recombinant DNA capable of expressing human ADF protein in yeast cells was constructed (FIGURE 5).

Vector pAM82 was cleaved with restriction enzyme Xho I. On the other hand, #20 cDNA was cleaved with restriction enzyme EcoR I. Both were made blunt ends using T4 DNA polymerase. Thereafter they were ligated using T4 DNA ligase. The obtained recombinant DNA was introduced into E. coli HB 101 strain and an ampicillin-resistant strain was selected. Plasmid DNA was extracted from the obtained strain and a cleavage test was conducted with a restriction enzyme, whereby plasmid pAM82 ADF-1 having inserted therein the human ADF cDNA downstream the Pho 5 promoter of pAM82 in the positive direction was selected.

Next, the obtained plasmid pAM82ADF-1 was introduced into Saccharomyces cerevisiae AH22 as follows.

Cells of Saccharomyces cerevisiae AH22 (leu 2, his 4, can 1, cir +) was shake cultured aerobically with shaking in 100 ml of YPD medium (1% yeast extract, 2% polypeptone, 2% glucose, pH 5.3) charged in a sakaguchi flask of 500 ml at 30°C. Centrifugation was performed at 3500 rpm for 5 minutes at the medium stage during the exponential growth phase (absorbancy at 610 nm, 0.8) to collect the cells. The cells were washed once with TE buffer (1 mM Tris-HCl, pH 7.5, 0.1 mM EDTA) and suspended in 5 ml of the same buffer. 0.5 ml of the cell suspension was taken in a test tube and an equal amount of 0.2 M $CH_3COOLi$ or 1.0 M CsCl was added to the suspension. Then, the suspension was shaken at 30°C for an hour. The cells in the suspension were centrifuged at 3500 rpm for 5 minutes. After washing once with 1 M sorbitol, the cells were again suspended in 1 ml of Zymolyase 5000 solution (1 M sorbitol, 0.1 M citrate buffer-10 mM EDTA-0.4 mg/ml Zymolyase 5000). The suspension was gently shaken at 37°C for an hour to prepare protoplast. The protoplast was washed 3 times with 1 M sorbitol and resuspended in 1.0 ml of a solution of 1 M sorbitol-10 mM $CaCl_2$-10mM Tris-HCl (pH 7.5). Plasmid DNA (pAM82ADF-1) was added to the protoplast suspension in 20 $\mu$g/ml. The mixture was incubated at room temperature for 5 minutes. Then, 5 ml of a solution of 40% polyethylene glycol-10 mM $CaCl_2$-10mM Tris-HCl (pH 7.5) was added to the mixture. Ten minutes after, the mixture was centrifuged to precipitate the protoplast. The precipitates were again suspended in 5 ml of a solution of 1 M sorbitol-10 mM $CaCl_2$-10mM Tris-HCl (pH 7.5). A part of the suspension, 0.2 ml, was added to 10 ml of reproduced agar medium and developed over the minimum agar plate (pH 5.3) composed of 0.1 M sorbitol, 0.67% Difco yeast nitrogen base, 2% glucose amino acid mixture solution (containing 5 mg/dl each of L-glutamic acid, L-aspartic acid, L-alanine, L-proline, L-threonine, L-serine, L-tryptophan, L-isoleucine, L-valine, L-phenylalanine, L-tyrosine, L-methionine, glycine, L-glutamine, L-asparagine, L-lysine, L-cysteine, L-histidine and L-arginine) and 2% agar thereby to select $Leu^+$ transformant.

Saccharomyces cerevisiae AH22 (FERM BP-1886)

containing this plasmid, pAM82 ADF-1 was aerobically cultured in 100 ml of high-phosphate Burkholder minimum medium (containing 20.0 g/l glucose, 2.0 g/l asparagine, 1.5 g/dl $KH_2PO_4$, 2.0 g/l $(NH_4)SO_4$, 0.5 g/l $MgSO_4.7H_2O$, 0.33 g/l $CaCl_2.2H_2O$, 0.1 mg/l KI, 0.25 mg/l $FeSO_4.7H_2O$, 0.04 mg/l $MnSO_4.4H_2O$, 0.02 mg/l $(NH_4)_6Mo_7O_{24}.4H_2O$, 0.60 mg/l $H_3BO_3$, 0.04 mg/l $CuSO_4 5H_2O$, 0.31 mg/l $ZnSO_4.7H_2O$, 0.20 mg/l thiamine hydrochloride, 10.0 mg/l inositol, 0.2 mg/l calcium panthothenate, 0.2 mg/l pyridoxine hydrochloride, 0.05 mg/ml p-aminobenzoic acid, 9.20 mg/l nicotinic acid and 2.0 $\gamma$/l biotin, pH 5.3) supplemented with histidine (20 $\mu$g/ml). When the cell density reached 0.4 in absorbancy at 610 nm, the sample was withdrawn, centrifuged and suspended in 100 ml of low phosphate minimum medium (containing 20.0 g/l glucose, 2.0 g/l asparagine, 1.5 g/dl KCl, 2.0 g/l $(NH_4)_2SO_4$, 0.5 g/l $MgSO_4.7H_2O$, 0.33 g/l $CaCl_2.2H_2O$, 0.1 mg/l KI, 0.25 mg/l $FeSO_4.7H_2O$, 0.04 mg/l $MnSO_4.4H_2O$, 0.02 mg/l $(NH_4)_6Mo_7O_{24}.4H_2O$, 0.60 mg/l $H_3BO_3$, 0.04 mg/l $CuSO_4.5H_2O$, 0.31 mg/l $ZnSO_4.7H_2 0$, 0.20 mg/l thiamine hydrochloride, 10.0 mg/l inositol, 0.2 mg/l calcium panthothenate, 0.2 mg/l pyridoxine hydrochloride, 0.05 mg/ml p-aminobenzoic acid, 9.20 mg/l nicotinic acid and 2.0 $\gamma$/l biotin, pH 5.3) to cause induction. A sample for control was treated in a similar manner except that it was not moved to the low-phosphate medium on the way. When the cell density reached 0.8 in absorbancy at 610 nm, 100 ml each of the samples in the induced culture solution and the culture solution which was not induced was centrifuged.

The obtained cells were suspended in 5 ml of a solution containing Zymolyase 5000 (100 $\mu$g/ml), 1.2 M sorbitol, 50 mM phosphate (pH 7.2) and 14 mM 2-mercaptoethanol. The suspension was incubated at 30°C for 30 minutes. By centrifugation, protoplast was collected and lysed in 1 ml of 0.1% Triton X-100/50 mM phosphate (pH 7.2) and 1 mM phenylmethylsulfonyl fluoride. The lysate was centrifuged at 11000 rpm for 20 minutes to make clear.

Using the lysate which was made clear, immunoblotting and the measurement of ADF activity and thioredoxin activity were performed. Expression of the protein having a molecular weight of about 13,000 capable of reacting with anti-human ADF antibody and expression of the ADF activity of 5 x $10^3$ U/ml and 5 x $10^{-3}$ U/ml of the thioredoxin activity were noted in the low-phosphate induced sample. To the contrary, neiter expression of human ADF protein nor expression of the ADF activity and thioredoxin activity was noted in the control sample which was cultured in the high-phosphate induced sample alone.

Example 8 Production of human ADF protein using monkey COS cells as host

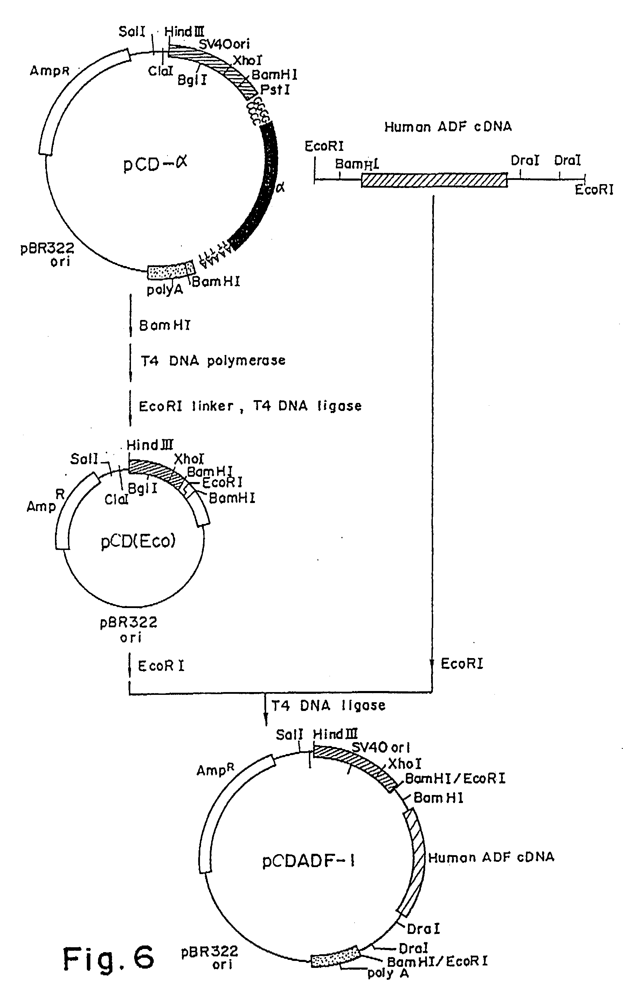

Using Okayama-Berg expression vector pCD $\alpha$ (H. Okayama and P. Berg., Mol. Cell. Biol., 3, 280-289 (1983)) having SV 40 promoter and #20 cDNA obtained in Example 3, recombinant DNA capable of expressing human ADF in monkey COS cells was constructed (FIGURE 6).

(a) Vector pCD $\alpha$ was cleaved with restriction enzyme BamH I. After the ends were made blunt using T4 DNA polymerase, EcoR I linker was ligated using T4 DNA ligase (pcD(Eco)). Next, #20 cDNA and pCD-

(Eco) were cleaved with restriction enzyme EcoR I. Both were made blunt ends using T4 DNA polymerase. Thereafter they were ligated using T4 DNA ligase. The obtained recombinant DNA was introduced into E. coli HB 101 strain and an ampicillin-resistant strain was selected. Plasmid DNA was extracted from the obtained strain and a cleavage test was conducted with restriction enzyme, whereby plasmid pCDADF-1 having inserted therein the human ADF cDNA downstream the SV 40 promoter of pAM82 in the positive direction was selected.

(b) Infection of plasmid DNA in monkey COS-7 cells

Plasmid pCDADF-1 was amplified by culture using E. coli and purified by CsCl ultracentrifugation, which was converted to DNA for monkey cell transfection.

COS-7 cells were suspended in 10% FCS-containing RPMI in $1 \times 10^5$/min. 3 ml of the suspension was cultured in a Petri dish having a diameter of 6 cm at 37°C overnight in a 5% carbon dioxide gas incubator.

The culture supernatant was removed and 3 ml of fresh RPMI containing 10% FCS was added thereto followed by culture at 37°C for 2 hours in the 5% carbon dioxide gas incubator. After the culture, the culture supernatant was removed. After the cells were washed with 2.5 ml of TBS (25 mM Tris-HCl, pH 7.5, 130 mM NaCl, 5 mM KCl, 0.6 mM $Na_2HPO_4$), 1 ml of DEAE-dextran-TBS solution (500 $\mu$g/ml DEAE-dextran, 25 mM Tris-HCl, pH 7.5, 130 mM NaCl, 5 mM KCl, 0.6 mM $Na_2HPO_4$), 0.7 mM $CaCl_2$, 0.5 mM $MgCl_2$) containing 2 $\mu$g of plasmid pcDADF-1 followed by culture at 37°C for 2 hours in the 5% carbon dioxide gas incubator. After the culture, the culture supernatant was removed and 2.5 ml of TBS containing 150 $\mu$M chloroquinone was added. After incubation was continued at 37°C for 5 hours in the 5% carbon dioxide gas incubator, the supernatant was removed and the cells were washed twice with 2.5 ml of TBS. 3 ml of 10% FCS-containing RPMI was added thereto followed by culture at 37°C overnight in the 5% carbon dioxide gas incubator. After the culture, the culture supernatant was recovered. After the supernatant was concentrated 10-fold using a centrifugal ultrafiltration concentrator, Centricon-10 (Amicon), immunoblotting and the measurement of ADF activity and thioredoxin activity were performed.

Expression of the protein having a molecular weight of about 13,000 capable of reacting with anti-human ADF antibody was noted in the culture supernatant of monkey COS-7 cells in which pCDADF-1 had been introduced. Further expression of the ADF activity of $5 \times 10^3$ u/ml and $5 \times 10^{-3}$ u/ml of the thioredoxin activity were noted.

Obviously, numerous modifications and variations of the present invention are possible in light of the above teachings. It is therefore to be understood that within the scope of the appended claims, the invention may be practiced otherwise than as specifically described herein.

**Claims**

1. A recombinant human adult leukemia cell derived factor, human ADF-polypeptide, having thioredoxin and ADF-activity and being selected from the following groups (a) to (d):

(a) a polypeptide, having the amino acid sequence

```
1
Val-Lys-Gln-Ile-Glu-Ser-Lys-Thr-Ala-Phe-Gln-Glu-Ala-Leu-Asp-Ala-Ala-Gly-Asp-Lys-Leu-Val-

Val-Val-Asp-Phe-Ser-Ala-Thr-Trp-Cys-Gly-Pro-

Cys-Lys-Met-Ile-Lys-Pro-Phe-Phe-His-Ser-Leu-
                            50
Ser-Glu-Lys-Tyr-Ser-Asn-Val-Ile-Phe-Leu-Glu-

Val-Asp-Val-Asp-Asp-Cys-Gln-Asp-Val-Ala-Ser-

Glu-Cys-Glu-Val-Lys-Cys-Met-Pro-Thr-Phe-Gln-

Phe-Phe-Lys-Lys-Gly-Gln-Lys-Val-Gly-Glu-Phe-

Ser-Gly-Ala-Asn-Lys-Glu-Lys-Leu-Glu-Ala-Thr-
100
Ile-Asn-Glu-Leu-Val,
```

(b) a polypeptide which in respect to (a) is deficient in one or more amino acids;
(c) a fusion polypeptide comprising a polypeptide according to (a) and additional amino acids in which the additional connected wino acids do not interfere with the biological activity or may be easily eliminated;
(d) a polypeptide which is an allelic derivative of a polypeptide according to any of (a), (b) or (c).

2. A recombinant human ADF polypeptide according to claim 1 wherein Met is added to the N-terminal of the recombinant human ADF polypeptide.

3. A DNA sequence coding for the recombinant human ADF polypeptide according to claim 1 or claim 2.

4. A DNA-sequence according to claim 3 which has the following base sequence (a):

```
1
GTGAAGC AGATCGAGAG CAAGACTGCT TTTCAGGAAG

CCTTGGACGC TGCAGGTGAT AAACTTGTAG TAGTTGACTT
                          100
CTCAGCCACG TGGTGTGGGC CTTGCAAAAT GATCAAGCCT

TTCTTTCATT CCCTCTCTGA AAAGTATTCC AACGTGATAT

TCCTTGAAGT AGATGTGGAT GACTGTCAGG ATGTTGCTTC
200
AGAGTGTGAA GTCAAATGCA TGCCAACATT CCAGTTTTTT

AAGAAGGGAC AAAAGGTGGG TGAATTTTCT GGAGCCAATA
                          300
AGGAAAAGCT TGAAGCCACC ATTAATGAAT TAGTC.
```

20

**5.** A DNA sequence according to claim 3 which has the following base sequence (b):

```
1
ATGGTGAAGC AGATCGAGAG CAAGACTGCT TTTCAGGAAG

CCTTGGACGC TGCAGGTGAT AAACTTGTAG TAGTTGACTT
                    100
CTCAGCCACG TGGTGTGGGC CTTGCAAAAT GATCAAGCCT

TTCTTTCATT CCCTCTCTGA AAAGTATTCC AACGTGATAT
                                      200
TCCTTGAAGT AGATGTGGAT GACTGTCAGG ATGTTGCTTC

AGAGTGTGAA GTCAAATGCA TGCCAACATT CCAGTTTTTT

AAGAAGGGAC AAAAGGTGGG TGAATTTTCT GGAGCCAATA
                    300
AGGAAAAGCT TGAAGCCACC ATTAATGAAT TAGTC.
```

**6.** A plasmid having incorporated therein a DNA sequence of any of the claims 3 to 5.

**7.** A plasmid according to claim 6, wherein the DNA sequence is incorporated operationally downstream of a promoter sequence.

**8.** A prokaryotic or eukaryotic cell transformed by a plasmid according to claim 6 or claim 7.

**9.** A cell according to claim 8, wherein the prokaryotic cell is Escherichia coli.

**10.** A cell according to claim 8, wherein the eukaryotic cell is Saccharomyces cerevisiae.

**11.** A cell according to claim 8, wherein the eukaryotic cell is a monkey COS cell.

**12.** A method of producing recombinant human ADF which comprises culturing in a medium a prokaryotic or eukaryotic cell as claimed in any of the claims 8, 9, 10, or 11 to produce a recombinant human ADF polypeptide and collecting said recombinant human ADF polypeptide from the medium.

**13.** A pharmaceutical composition comprising as an effective ingredient recombinant human ADF according to claim 1 or claim 2.

**14.** An immunotherapeutic composition comprising as an active ingredient recombinant human ADF according to claim 1 or claim 2.

**15.** A pharmaceutical composition for the treatment of inflammatory diseases, comprising as an effective ingredient recombinant human ADF according to claim 1 or claim 2.

**Patentansprüche**

**1.** Von humanen adulten Leukämiezellen abgeleiteter rekombinanter Faktor, humanes ADF-Polypeptid, das Thioredoxin- und ADF-Aktivität aufweist und eines der folgenden Polypeptide ist:

(a) ein Polypeptid mit der nachstehenden Aminosäuresequenz

```
                 1
    (a)   Val-Lys-Gln-Ile-Glu-Ser-Lys-Thr-Ala-Phe-Gln-Glu-Ala-Leu-Asp-Ala-Ala-Gly-Asp-Lys-Leu-Val-

          Val-Val-Asp-Phe-Ser-Ala-Thr-Trp-Cys-Gly-Pro-

          Cys-Lys-Met-Ile-Lys-Pro-Phe-Phe-His-Ser-Leu-
                               50
          Ser-Glu-Lys-Tyr-Ser-Asn-Val-Ile-Phe-Leu-Glu-

          Val-Asp-Val-Asp-Asp-Cys-Gln-Asp-Val-Ala-Ser-

          Glu-Cys-Glu-Val-Lys-Cys-Met-Pro-Thr-Phe-Gln-

          Phe-Phe-Lys-Lys-Gly-Gln-Lys-Val-Gly-Glu-Phe-

          Ser-Gly-Ala-Asn-Lys-Glu-Lys-Leu-Glu-Ala-Thr-
          100
          Ile-Asn-Glu-Leu-Val,
```

(b) ein Polypeptid, dem gegenüber (a) eine oder mehr Aminosäuren fehlen,

(c) ein Fusionspolypeptid, welches ein Polypeptid gemäß (a) und zusätzliche Aminosäuren enthält, in dem die zusätzlich verknüpften Aminosäuren die biologische Aktivität nicht beeinträchtigen oder leicht entfernt werden können,

(d) ein Polypeptid, welches ein allelisches Derivat eines Polypeptids gemäß einem der Punkte (a), (b) oder (c) ist.

2.  Rekombinantes humanes ADF-Polypeptid nach Anspruch 1, in dem Met an den N-Terminus des rekombinanten humanen ADF-Polypeptids gebunden ist.

3.  Das rekombinante humane ADF-Polypeptid nach Anspruch 1 oder Anspruch 2 kodierende DNA-Sequenz.

4. DNA-Sequenz nach Anspruch 3, welche die folgende Basensequenz (a) aufweist:

```
     1
     GTGAAGC AGATCGAGAG CAAGACTGCT TTTCAGGAAG

     CCTTGGACGC TGCAGGTGAT AAACTTGTAG TAGTTGACTT
                                  100
     CTCAGCCACG TGGTGTGGGC CTTGCAAAAT GATCAAGCCT

     TTCTTTCATT CCCTCTCTGA AAAGTATTCC AACGTGATAT

     TCCTTGAAGT AGATGTGGAT GACTGTCAGG ATGTTGCTTC
      200
     AGAGTGTGAA GTCAAATGCA TGCCAACATT CCAGTTTTTT

     AAGAAGGGAC AAAAGGTGGG TGAATTTTCT GGAGCCAATA
                                  300
     AGGAAAAGCT TGAAGCCACC ATTAATGAAT TAGTC.
```

5. DNA-Sequenz nach Anspruch 3, welche die folgende Basensequenz (b) aufweist:

```
     1
     ATGGTGAAGC AGATCGAGAG CAAGACTGCT TTTCAGGAAG

     CCTTGGACGC TGCAGGTGAT AAACTTGTAG TAGTTGACTT
                                  100
     CTCAGCCACG TGGTGTGGGC CTTGCAAAAT GATCAAGCCT

     TTCTTTCATT CCCTCTCTGA AAAGTATTCC AACGTGATAT
                                              200
     TCCTTGAAGT AGATGTGGAT GACTGTCAGG ATGTTGCTTC

     AGAGTGTGAA GTCAAATGCA TGCCAACATT CCAGTTTTTT

     AAGAAGGGAC AAAAGGTGGG TGAATTTTCT GGAGCCAATA
                                  300
     AGGAAAAGCT TGAAGCCACC ATTAATGAAT TAGTC.
```

6. Plasmid, in das eine DNA-Sequenz nach einem der Ansprüche 3 bis 5 inseriert ist.

7. Plasmid nach Anspruch 6, wobei die DNA-Sequenz funktionsfähig stromabwärts einer Promotorsequenz inseriert ist.

8. Mit einem Plasmid nach Anspruch 6 oder Anspruch 7 transformierte prokaryotische oder eukaryotische Zelle.

9. Zelle nach Anspruch 8, wobei die prokaryotische Zelle Escherichia coli ist.

10. Zelle nach Anspruch 8, wobei die eukaryotische Zelle Saccharomyces cerevisiae ist.

**11.** Zelle nach Anspruch 8, wobei die eukaryotische Zelle eine Affen-COS-Zelle ist.

**12.** Verfahren zur Herstellung von rekombinantem humanem ADF, bei dem in einem Medium eine prokaryotische oder eukaryotische Zelle gemäß einem der Ansprüche 8, 9, 10 oder 11 unter Bildung eines rekombinanten humanen ADF-Polypeptids gezüchtet wird und das rekombinante humane ADF-Polypeptid aus dem Medium gewonnen wird.

**13.** Arzneimittelzusammensetzung, die als Wirkstoff rekombinantes humanes ADF nach Anspruch 1 oder Anspruch 2 enthält.

**14.** Immuntherapeutische Zusammensetzung, die als Wirkstoff rekombinantes humanes ADF nach Anspruch 1 oder Anspruch 2 enthält.

**15.** Arzneimittelzusammensetzung zur Behandlung von entzündlichen Erkrankungen, die als Wirkstoff rekombinantes humanes ADF nach Anspruch 1 oder Anspruch 2 enthält.

**Revendications**

**1.** Polypeptide recombiné d'ADF humain, facteur dérivé de cellules de la leucémie humaine de l'adulte, qui a une activité de thiorédoxine et d'ADF et qui est choisi dans les groupes (a) à (d) suivants:
   (a) un polypeptide ayant la séquence d'aminoacides

```
    1
Val-Lys-Gln-Ile-Glu-Ser-Lys-Thr-Ala-Phe-Gln-Glu-Ala-Leu-Asp-Ala-Ala-Gly-Asp-Lys-Leu-Val-

Val-Val-Asp-Phe-Ser-Ala-Thr-Trp-Cys-Gly-Pro-

Cys-Lys-Met-Ile-Lys-Pro-Phe-Phe-His-Ser-Leu-
                           50
Ser-Glu-Lys-Tyr-Ser-Asn-Val-Ile-Phe-Leu-Glu-

Val-Asp-Val-Asp-Asp-Cys-Gln-Asp-Val-Ala-Ser-

Glu-Cys-Glu-Val-Lys-Cys-Met-Pro-Thr-Phe-Gln-

Phe-Phe-Lys-Lys-Gly-Gln-Lys-Val-Gly-Glu-Phe-

Ser-Gly-Ala-Asn-Lys-Glu-Lys-Leu-Glu-Ala-Thr-
100
Ile-Asn-Glu-Leu-Val,
```

   (b) un polypeptide dans lequel, par rapport à (a), manquent un ou plusieurs aminoacides;
   (c) un polypeptide de fusion comprenant un polypeptide selon (a) et des aminoacides supplémentaires dans lequel les aminoacides reliés supplémentaires ne gênent pas l'activité biologique d'ADF ou s'éliminent facilement;
   (d) un polypeptide qui est un dérivé allélique d'un polypeptide correspondant à l'un quelconque des (a), (b) ou (c).

**2.** Polypeptide d'ADF humain recombiné selon la revendication 1, dans lequel un Met est ajouté à l'extrémité N-terminale du polypeptide d'ADF humain recombiné.

**3.** Séquence d'ADN codant pour le polypeptide d'ADF humain recombiné selon la revendication 1 ou la revendication 2.

4.   Séquence d'ADN selon la revendication 3, qui a la séquence de bases (a) suivante:

```
1
GTGAAGC AGATCGAGAG CAAGACTGCT TTTCAGGAAG

CCTTGGACGC TGCAGGTGAT AAACTTGTAG TAGTTGACTT
                        100
CTCAGCCACG TGGTGTGGGC CTTGCAAAAT GATCAAGCCT

TTCTTTCATT CCCTCTCTGA AAAGTATTCC AACGTGATAT

TCCTTGAAGT AGATGTGGAT GACTGTCAGG ATGTTGCTTC
   200
AGAGTGTGAA GTCAAATGCA TGCCAACATT CCAGTTTTTT

AAGAAGGGAC AAAAGGTGGG TGAATTTTCT GGAGCCAATA
                                 300
AGGAAAAGCT TGAAGCCACC ATTAATGAAT TAGTC.
```

5.   Séquence d'ADN selon la revendication 3, qui a la séquence de bases (b) suivante:

```
1
ATGGTGAAGC AGATCGAGAG CAAGACTGCT TTTCAGGAAG

CCTTGGACGC TGCAGGTGAT AAACTTGTAG TAGTTGACTT
                        100
CTCAGCCACG TGGTGTGGGC CTTGCAAAAT GATCAAGCCT

TTCTTTCATT CCCTCTCTGA AAAGTATTCC AACGTGATAT
                                      200
TCCTTGAAGT AGATGTGGAT GACTGTCAGG ATGTTGCTTC

AGAGTGTGAA GTCAAATGCA TGCCAACATT CCAGTTTTTT

AAGAAGGGAC AAAAGGTGGG TGAATTTTCT GGAGCCAATA
                                 300
AGGAAAAGCT TGAAGCCACC ATTAATGAAT TAGTC.
```

6.   Plasmide dans lequel est incorporée une séquence d'ADN selon l'une quelconque des revendications 3 à 5.

7.   Plasmide selon la revendication 6, dans lequel la séquence d'ADN est incorporée de façon opérationnelle en aval d'une séquence de promoteur.

8.   Cellule procaryote ou eucaryote transformée par un plasmide selon la revendication 6 ou la revendication 7.

9.   Cellule selon la revendication 8, dans laquelle la cellule procaryote est *Escherichia coli*.

10.  Cellule selon la revendication 8, dans laquelle la cellule eucaryote est *Saccharomyces cerevisiae*.

11.  Cellule selon la revendication 8, dans laquelle la cellule eucaryote est une cellule COS de singe.

**12.** Procédé de production d'ADF humain recombiné, dans lequel on cultive dans un milieu une cellule procaryote ou eucaryote selon l'une quelconque des revendications 8, 9, 10 ou 11 pour produire un polypeptide d'ADF humain recombiné et on recueille ledit polypeptide d'ADF humain recombiné à partir du milieu.

**13.** Composition pharmaceutique comprenant comme ingrédient actif de l'ADF humain recombiné selon la revendication 1 ou la revendication 2.

**14.** Composition immunothérapeutique comprenant comme ingrédient actif de l'ADF humain recombiné selon la revendication 1 ou la revendication 2.

**15.** Composition pharmaceutique pour le traitement de maladies inflammatoires, comprenant comme ingrédient actif de l'ADF humain recombiné selon la revendication 1 ou la revendication 2.

Fig. I

G AGT.CTT.GAA.GCT.CTG.TTT.GGT.GCT.TTG.GAT.CCA.TTT.CCA.TCG.GTC.CTT.ACA.GCC.GCT.CGT

CAG.ACT.CCA.GCA.GCC.AAG.ATG.GTG.AAG.CAG.ATC.GAG.AGC.AAG.ACT.GCT.TTT.CAG.GAA.GCC
                              Met-Val-Lys-Gln-Ile-Glu-Ser-Lys-Thr-Ala-Phe-Gln-Glu-Ala

TTG.GAC.GCT.GCA.GGT.GAT.AAA.CTT.GTA.GTA.GTT.GAC.TTC.TCA.GCC.ACG.TGG.TGT.GGG.CCT
Leu-Asp-Ala-Ala-Gly-Asp-Lys-Leu-Val-Val-Val-Asp-Phe-Ser-Ala-Thr-Trp-Cys-Gly-Pro

TGC.AAA.ATG.ATC.AAG.CCT.TTC.TTT.CAT.TCC.CTC.TCT.GAA.AAG.TAT.TCC.AAC.GTG.ATA.TTC
Cys-Lys-Met-Ile-Lys-Pro-Phe-Phe-His-Ser-Leu-Ser-Glu-Lys-Tyr-Ser-Asn-Val-Ile-Phe

CTT.GAA.GTA.GAT.GTG.GAT.GAC.TGT.CAG.GAT.GTT.GCT.TCA.GAG.TGT.GAA.GTC.AAA.TGC.ATG
Leu-Glu-Val-Asp-Val-Asp-Asp-Cys-Gln-Asp-Val-Ala-Ser-Glu-Cys-Glu-Val-Lys-Cys-Met

CCA.ACA.TTC.CAG.TTT.TTT.AAG.AAG.GGA.CAA.AAG.GTG.GGT.GAA.TTT.TCT.GGA.GCC.AAT.AAG
Pro-Thr-Phe-Gln-Phe-Phe-Lys-Lys-Gly-Gln-Lys-Val-Gly-Glu-Phe-Ser-Gly-Ala-Asn-Lys

GAA.AAG.CTT.GAA.GCC.ACC.ATT.AAT.GAA.TTA.GTC.TAA.TCA.TGT.TTC.TGA.AAA.TAT.AAC.CAG
Glu-Lys-Leu-Glu-Ala-Thr-Ile-Asn-Glu-Leu-Val-***

CCA.TTG.AGC.TAT.TTA.AAA.CTT.GTA.ATT.TTT.TTA.ATT.TAC.AAA.AAT.ATA.AAA.TAT.GAA.GAC

ATA.AAC.CCA.GTT.GCC.ATC.TGC.GTG.ACA.ATA.AAA.CAT.TAA.TGC.TAA.CAC.TTT.TTA.AAA.CCG.

TCT.CAT.GTC.TGA.ATA.GCT.TTC.AAA.ATA.AAT.GTG.AAA.TGG.TC

# Fig. 2

EP 0 299 206 B1

Fig. 3

29

EP 0 299 206 B1

CLOSTRIPAIN
Kallikrein
↓

Ala Thr Glu Phe Pro Ser Leu Glu Ala Leu Phe Gly Ala Leu Asp Leu Phe Arg Val Lys Glu Ile Glu

····GCC ACA GAA TTC CCG AGT CTT GAA GCT CTG TTT GGT GCT TTG GAT CTC TTC AGA GTG AAG CAG ATC GAG·······

····CGG TGT CTT AAG GGC TCA GAA CTT CGA GAC AAA CCA CGA AAC CTA GAG AAG TCT CAC TTC GTC TAG GTA·······

HIL-2 ——→ EcoRI ——————————— Human ADF cDNA ————————————→ BamHI ———————— Adapter ————————→ TaqI ——→
cDNA      (SacI)

                                                                                    ⌐ Human ADFcDNA

(SacI)/EcoRI
(BamHI)
Adapter
HIL-2cDNA        Taq I
NcoI
                Human
                ADFcDNA
trp P/O

pTfADF-2         (DraI/StuI)
                 BamHI

                 trp A terminator
                 PvuⅡ
        amp

Fig. 4

Fig. 5

Fig. 6